# EUROPÄISCHE PATENTSCHRIFT

(11) **EP 0 989 520 B1**
(45) Veröffentlichungstag und Bekanntmachung des Hinweises auf die Patenterteilung: **01.05.2013**
(21) Anmeldenummer: 99203003.1
(22) Anmeldetag: 15.09.1999
(51) Int. Cl.: G06T 11/00

(54) **Computertomographie-Verfahren mit kegelförmigem Strahlenbündel**
Computer tomography method with cone beam
Procédé de tomographie par calculateur avec faisceau en forme conique

(30) Priorität: 24.09.1998 DE 19843812
(43) Veröffentlichungstag der Anmeldung: 29.03.2000
(73) Patentinhaber: Philips Intellectual Property & Standards GmbH, 20099 Hamburg (DE); Koninklijke Philips Electronics N.V., 5621 BA Eindhoven (NL)
(72) Erfinder: Grass, Michael, Dr., Habsburgerallee 11, 52064 Aachen (DE); Proksa, Roland, Habsburgerallee 11, 52064 Aachen (DE)
(74) Vertreter: Volmer, Georg

(56) Entgegenhaltungen:
- SILVER M D: "High-helical-pitch, cone-beam computed tomography" PHYSICS IN MEDICINE AND BIOLOGY IOP PUBLISHING UK, Bd. 43, Nr. 4, April 1998 (1998-04), Seiten 847-855, XP002604256 ISSN: 0031-9155
- DANIELSSON P-E ET AL: "Towards Exact 3D-Reconstruction for Helical Cone-Beam Scanning of Long Objects. A New Detector Arrangement and a New Completeness Condition" PROCEEDINGS OF THE INTERNATIONAL MEETING ON FULLYTHREE-DIMENSIONAL IMAGE RECONSTRUCTION IN RADIOLOGY ANDNUCLEAR MEDICINE, XX, XX, 27. Juni 1997 (1997-06-27), Seiten 1-15, XP002192547
- FELDKAMP L A ET AL: "Practical cone-beam algorithm" JOURNAL OF THE OPTICAL SOCIETY OF AMERICA A, OPTICAL SOCIETY OF AMERICA, US, Bd. 1, Nr. 6, 1. Juni 1984 (1984-06-01), Seiten 612-619, XP002085783 ISSN: 1084-7529

## Beschreibung

Die Erfindung betrifft ein Computertomographie-Verfahren mit den Schritten
- Erzeugen eines kegelförmigen, einen Untersuchungsbereich bzw. ein darin befindliches Objekt durchsetzenden Strahlenbündels,
- Erzeugung einer eine Rotation um eine Rotationsachse umfassenden Relativbewegung zwischen dem Strahlenbündel und dem Untersuchungsbereich bzw. dem Objekt,
- Akquisition von Meßdaten, die von der Intensität in dem Strahlenbündel jenseits des Untersuchungsbereiches abhängen während der Relativbewegung,
- Rekonstruktion der räumlichen Verteilung der Absorption innerhalb des Untersuchungsbereiches aus den von der Detektoreinheit akquirierten Meßdaten.

Außerdem bezieht sich die Erfindung auf einen Computertomographen zur Durchführung dieses Verfahrens.

Als "kegelförmig" wird ein Strahlenbündel bezeichnet, das in zwei zueinander senkrechten Richtungen eine endliche Ausdehnung hat und das von einer Detektoreinheit erfaßt wird, die die durch den Untersuchungsbereich geschwächte Intensität des Strahlenbündels ortsauflösend in diesen zwei Richtungen messen kann. Ein solches Verfahren ist aus einer Veröffentlichung von L.A. Feldkamp et al "Practical Cone Beam Algorithms", Journal of Optical Soc. Am.A,Vol.1,No.6/pp 612-619, 1984, bekannt.

Ein grundsätzlicher Nachteil von CT-Verfahren (CT=Computertomographie) mit kegelförmigem Strahlenbündel besteht darin, daß während der Relativbewegung zwischen Strahlenquelle und Untersuchungsbereich einige Voxel (Volumenelemente) in dem Untersuchungsbereich nur zeitweise von der Strahlung getroffen werden und daß die Absorption in diesen Voxeln aus den von der Detektoreinheit akquirierten Meßdaten nicht rekonstruiert werden kann. Der Teil des Untersuchungsbereichs, in dem die räumliche Absorptionsverteilung rekonstruiert werden kann, ist also stets kleiner als der von der Strahlung getroffene Teil.

Bei dem bekannten Verfahren wird ein Rekonstruktionsalgorithmus angewandt, mit dem die Absorption innerhalb des während der gesamten Relativbewegung von Strahlung getroffenen rotationssymmetrischen Bereiches mit einer einem Diskus ähnlichen Form rekonstruiert wird, wobei sich in der Praxis die Rekonstruktion auf eine ebene Scheibe innerhalb dieses Bereichs beschränken dürfte. Dieses bekannte Verfahren geht von einer kreisförmigen Relativbewegung aus.

Michael D. Silver, "High-helical-pitch, cone-beam computed tomography", PHYSICS IN MEDICINE AND BIOLOGY Bd. 43, Nr. 4, April 1998, Seiten 847-855, offenbart ein tomographisches Rekonstruktionsverfahren, basierend auf dem Feldkamp-Algorithmus, bei welchem zusätzlich der verwendete Winkelbereich für jedes Pixel der Bildebene abhängig von dessen Bestrahlungsbereich variiert wird. Start- und Endwinkel sind Funktionen der Pixelposition. Der dabei überstrichene Winkelbereich erfüllt die Vollständigkeitsbedingung Δβ ≥ 180° + 2γ wobei γ der Öffnungswinkel des Strahlenbündels ist.

Es gibt aber auch CT-Verfahren mit einer helixförmigen Relativbewegung. Bei diesem Verfahren wird die Absorption in den bei Beginn oder am Ende der Relativbewegung im Strahlenbündel befindlichen Voxeln nicht rekonstruiert.

Aufgabe der vorliegenden Erfindung ist es, den Bereich in dem die Absorptionsverteilung rekonstruiert wird, zu vergrößern. Diese Aufgabe wird erfindungsgemäß gelöst durch die Shritte zur Rekonstruktion der räumlichen Verteilung der Absorption des kennzeichnenden Teils von Anspruch 1.

Die Erfindung basiert auf der Überlegung, daß die bekannten Verfahren zur Rekonstruktion immer nur einen Rekonstruktionsalgorithmus benutzen. Die Absorption wird dabei für jedes Voxel mit Rechenschritten von gleicher Art und gleicher Zahl rekonstruiert. Jeder Rekonstruktionsalgorithmus ist an bestimmte Randbedingungen gebunden (z.B. die, daß alle Voxel in dem zu rekonstruierenden Volumen während der gesamten Relativbewegung von Strahlung getroffen worden sind), die nur in einem Teil des Gesamtvolumens (dem ersten Teilvolumen) erfüllt sind. Diese Randbedingungen sind zwar hinreichend, für die Rekonstruktion aber nicht notwendig, d.h. es gibt zusätzliche Voxel, die diese Randbedingung nicht erfüllen, jedoch weniger stringente Randbedingungen, die für eine Rekonstruktion - mit einem anderen Rekonstruktionsalgorithmus - hinreichen, auch wenn das Signal/Rauschverhältnis dabei ungünstiger sein kann. Diese Voxel befinden sich in einem anderen Teil des Gesamtvolumens (dem zweiten Teilvolumen).

Somit kann man den Rekonstruktionsbereich erweitern, indem man ein hybrides Rekonstruktionsverfahren mit einem ersten Rekonstruktionsalgorithmus in einem ersten Teilvolumen und einem zweiten Rekonstruktionsalgorithmus in einem zweiten (vom ersten verschiedenen) Teilvolumen anwendet. Dabei kann der zweite Rekonstruktions algorithmus durchaus Rechenschritte gleicher Art enthalten wie der erste, jedoch in einer unterschiedlichen Anzahl. Die Angabe "abweichender Rekonstruktionsalgorithmus" ist also in diesem Sinne breit zu interpretieren.

Anspruch 2 beschreibt eine Ausgestaltung, die bei einer kreisförmigenTrajektorie anwendbar ist (bei der also die Relativbewegung zwischen Strahlenquelle und Detektoreinheit einerseits und dem Untersuchungsbereich andrerseits die Form eines Kreises hat). Dabei ist das Zuordnungskriterium zu den beiden Teilvolumina durch den Bestrahlungswinkelbereich gegeben (das ist der Winkelbereich, den die (Parallel-) Projektion der Strahlen von der Strahlenquelle zu einem Voxel auf eine zur Rotationsachse senkrechte Ebene in dieser Ebene bedecken, bzw. den die Komponenten der Vektoren von der Strahlenquelle zu dem Voxel in der Rotationebene der Strahlenquelle überstreichen). Voxel mit einem Bestrahlungswinkelbereich von 360° (diese Voxel werden während der gesamten Relativbewegung von Strahlung getroffen) werden dem ersten Teilbereich zugeordnet und Voxel mit einem Bestrahlungswinkelbereich von mindestens 180° (aber weniger als 360°) dem zweiten Teilvolumen, das das erste Teilvolumen beiderseits begrenzt und das zur Rotationsachse senkrechte Seitenflächen aufweist.

Die Rekonstruktion der Absorption der Voxel in dem ersten Teilvolumen erfolgt dabei mit einem ersten Rekonstruktionsalgorithmus, der einen Rekonstruktionswinkelbereich von 360° ausnutzt (als Rekonstruktionswinkelbereich wird der Winkelbereich bezeichnet, den die zur Rekonstruktion herangezogenen (Parallel-) Projektionen der Strahlen von der Strahlenquelle zu einem Voxel auf eine zur Rotationsachse senkrechte Ebene in dieser Ebene bedecken.) Als Rekonstruktionsalgorithmus kann dabei beispielsweise der in der eingangs erwähnten Veröffentlichung beschriebene Algorithmus verwenden. Für das zweite Teilvolumen kann ein Rekonstruktionsalgorithmus verwendet werden, der mit einem Rekonstruktionswinkelbereich von 180° auskommt; bei den CT-Verfahren mit einem ebenen fächerförmigen Strahlenbündel werden bekanntlich auch Rekonstruktionsalgorithmen benutzt werden, die mit einem Rekonstruktionswinkelbereich von nur 180° arbeiten.

Die Ansprüche 3 und 4 beschreiben zwei Alternativen, wie man die Absorption in diesen Voxeln rekonstruieren kann. Gemäß Anspruch 3 werden nur Meßdaten aus einem Bestrahlungswinkelbereich von exakt 180° herangezogen. Gemäß Anspruch 4 werden alle Meßdaten herangezogen, die für Strahlen durch das betreffende Voxel ermittelt worden sind, jedoch werden die Beiträge von Strahlen, deren Pojektion das Voxel aus um 180° gegeneinander versetzten Richtung passiert, so gewichtet, daß ihr Gesamtgewicht so groß ist wie das eines Einzelstrahls (d.h. eines Strahls für eine Richtung, für die es in der Gegenrichtung keinen Strahl gibt). Die Rekonstruktion ist in diesem Fall einer Rekonstruktion mit einem Rekonstruktionswinkelbereich von 180° äquivalent, hat allerdings ein günstigeres Signal/Rausch-Verhältnis.

Wenn der mit einer kreisförmigen Trajektorie rekonstruierbare Teil des Untersuchungsbereichs nicht ausreicht, kann man den Untersuchungsbereich auf zwei nebeneinander liegenden kreisförmigen Abtastbahnen abtasten. Anspruch 5 beschreibt eine für diesen Fall geeignete Ausgestaltung des erfindungsgemäßen Verfahrens. Dabei ist symmetrisch zu den beiden Kreisen, auf denen die Relativbewegung erfolgt, ein (scheibenförmiger) Zwischenbereich vorgesehen, der ein drittes Teilvolumen bildet. Während die Absorption der Voxel in dem ersten und zweiten Teilvolumen so rekonstruiert werden kann, wie in Verbindung mit Anspruch 2 erläutert, kann das dritte Teilvolumen z.B. mit einem ART-Verfahren (ART=Algebraic Reconstruction Technique) rekonstruiert werden.

Anspruch 6 bezieht sich auf eine Ausgestaltung für eine helixförmige Relativbewegung zwischen dem Untersuchungsbereich und der Strahlenquelle bzw. der Detektoreinheit. In der Offenlegungs schrift DE 19 835 296 A1 ist ein Verfahren dieser Art beschrieben, bei dem sich für (einen Teil der) Voxel im Untersuchungsbereich ein Bestrahlungswinkelbereich von genau (2n+1)π ergibt, wobei n eine ganze Zahl ist. Mit dem bekannten Verfahren kann die Absorption in diesen Voxeln rekonstruiert werden. Die Absorption von Voxeln, die sich am Beginn oder am Ende der helixförmigen Relativbewegung innerhalb des Strahlenbündels befinden, ist mit dem in dieser die in diesem Dokument beschriebenen Rekonstruktionsverfahren nicht möglich, weil der Strahlungswinkelbereich für diese Voxel kleiner ist als (2n+1)π. Deshalb ist die Randbedingung für das in dem genannten Dokument beschriebene Rekonstruktionsverfahren verletzt, jedoch gibt es dabei Voxel mit einem Bestrahlungswinkelbereich von π oder mehr. Diese Voxel werden dem zweiten Teilvolumen zugeordnet, und ihre Absorption kann mit dem aus der WO 98/30980 bekannten Verfahren rekonstruiert werden, wenn man jeweils nur die Meßdaten für einen Bestrahlungswinkelbereich von exakt 180° heranzieht.

Ein Computertomograph zur Durchführung des erfindungsgemäßen Verfahrens ist in Anspruch 7 beschrieben.

Die Erfindung wird nachstehend anhand der Figuren näher erläutert. Es zeigen:
- Fig. 1: Einen Computertomographen bei dem die Erfindung anwendbar ist.
- Fig. 2A-2C: Die Lage der Teilvolumina bzw. einzelner Voxel auf der Seitenfläche eines Teilvolumens
- Fig. 3: Ein Ablaufdiagramm für ein erfindungsgemäßes Rekonstruktionsverfahren.
- Fig. 4: Die geometrischen Verhältnisse für ein Voxel innerhalb des zweiten Teilvolumens.
- Fig. 5a und 5b: Die Gewichtungsfaktoren, mit dem die verschiedenen Bestrahlungsrichtungen bei der Rekonstruktion der Absorption eines Voxels eingehen.
- Fig. 6: Die geometrischen Verhältnisse bei einer Abtastung des Untersuchungsbereichs auf zwei gegeneinander versetzten Kreisbahnen und
- Fig. 7: die geometrischen Verhältnisse bei einer helixförmigen Relativbewegung.

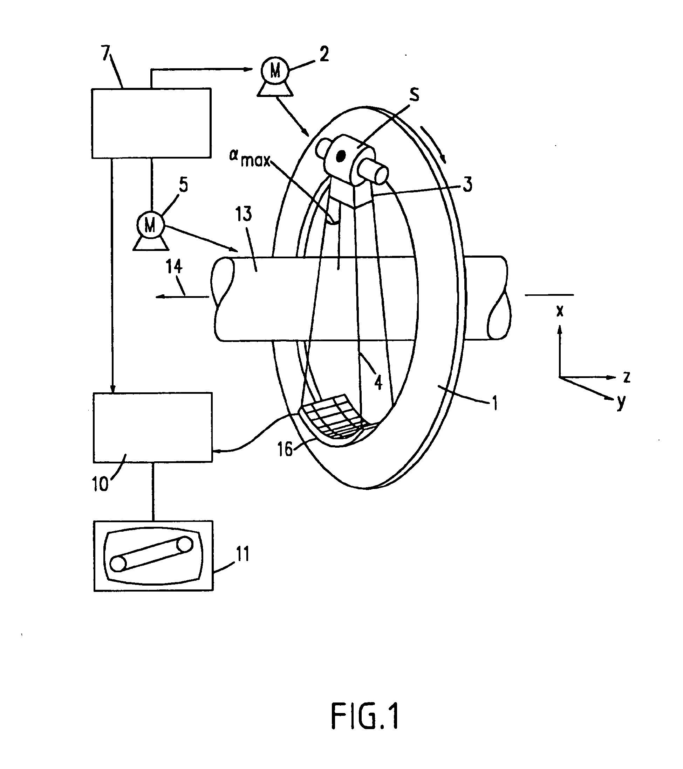

Der in Fig. 1 dargestellte Computertomograph umfaßt eine Gantry 1, die um eine parallel zur z-Richtung des in Fig. 1 dargestellten Koordinatensystems verlaufende Rotationsachse 14 rotieren kann. Dazu wird die Gantry von einem Motor 2 mit einer vorzugsweise konstanten, aber einstellbaren Winkelgeschwindigkeit angetrieben. An der Gantry ist eine Strahlenquelle S, beispielsweise ein Röntgenstrahler, befestigt. Dieser ist mit einer Kollimatoranordnung 3 versehen, die aus der von der Strahlenquelle S erzeugten Strahlung ein kegelförmiges Strahlenbündel 4 ausblendet, d.h. ein Strahlenbündel, das sowohl in z-Richtung als auch in einer dazu senkrechten Richtung (das heißt in der x,y-Ebene) eine von Null verschiedene, endliche Ausdehnung hat.

Das Strahlenbündel 4 durchdringt einen Untersuchungsbereich 13 in dem sich ein Patient auf einem Patientenlagerungstisch (beides nicht näher dargestellt) befinden kann. Der Untersuchungsbereich 13 hat die Form eines Zylinders, der im folgenden als Objektzylinder 13 bezeichnet wird. Nach dem Durchsetzen des Objektzylinders 13 trifft das Röntgenstrahlenbündel 4 auf eine an der Gantry 1 befestigte zweidimensionale Detektoreinheit 16, die eine Anzahl von Detektorzeilen mit jeweils einer Vielzahl von Detektorelementen umfaßt. Jedes Detektorelement liefert in jeder Position der Strahlenquelle einen Meßwert für einen Strahl aus dem Strahlenbündel 4. Die Detektoreinheit 16 kann auf einem Kreisbogen um die Rotationsachse 14 angeordnet sein, aber auch auf einem Kreisbogen um die Strahlenquelle S; sie kann aber auch eben sein.

Der mit αₘₐₓ bezeichnete Öffnungswinkel des Strahlenbündels 4 (als Öffnungswinkel ist der Winkel definiert, den ein in der x,y-Ebene am Rande liegender Strahl des Bündels 4 mit einer durch die Strahlenquelle S und die Rotationsachse 14 definierten Zentralstrahlebene einschließt) bestimmt dabei den Durchmesser des Objektzylinders 13, innerhalb dessen sich das zu untersuchende Objekt bei der Akquisition der Meßwerte befindet. Der Untersuchungsbereich 13 - bzw. das Objekt oder der Patientenlagerungstisch - kann mittels eines Motors 5 parallel zur Rotationsachse 14 bzw. zur z-Achse verschoben werden. Die Geschwindigkeit dieses Vorschubs in z-Richtung ist konstant und vorzugsweise einstellbar.

Die von der Detektoreinheit akquirierten Meßdaten werden einem Bildverarbeitungsrechner 10 zugeführt, der daraus die Absorptionsverteilung in einem Teil des Untersuchungsbereichs 13 rekonstruiert und z.B. auf einem Monitor 11 wiedergibt. Die beiden Motoren 2 und 5, der Bildverarbeitungsrechner 10, die Strahlenquelle S und der Transfer der Meßdaten von der Detektoreinheit zum Bildverarbeitungsrechner 10 werden von einer geeigneten Kontrolleinheit 7 gesteuert.

Wenn der Motor 5 für den Vorschub in z-Richtung stillsteht und der Motor 2 die Gantry 1 rotieren läßt, ergibt sich eine kreisförmige Abtastbewegung der Strahlenquelle S und der Detektoreinheit. Die Kontrolleinheit 7 kann die Motoren 2 und 5 aber auch gleichzeitig so aktivieren, daß das Verhältnis der Vorschubgeschwindigkeit des Untersuchungsbereichs 13 und die Winkelgeschwindigkeit der Gantry in einem konstanten Verhältnis stehen.

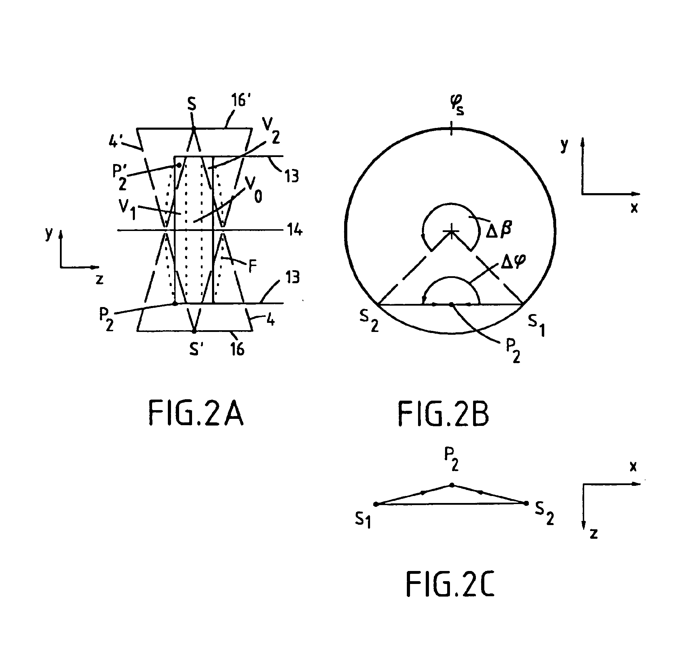

Fig. 2A zeigt die durch einen Punkt angedeutete Strahlenquelle S, die durch eine Linie symbolisierte Detektoreinheit 16 und das Strahlenbündel 4 in einer ersten Position bezüglich der Rotationsachse 14 und - durch die Bezugszeichen S', 16' und 4' angedeutet - in einer zweiten, demgegenüber um 180° versetzten Position. Außerdem ist in Fig. 2A der Untersuchungsbereich 13 angedeutet. Dabei sind der Deutlichkeit halber die Abmessungen in Richtung parallel zur Rotationsachse in einem größer Maßstab dargestellt als senkrecht dazu. Die Absorption in allen Voxeln, die sich innerhalb des - annähernd diskusförmigen - Teilvolumens V₁ des Untersuchungsbereichs 13 befinden, das von den Strahlenbündeln 4 und 4' - und den in allen andern Strahlenquellenpositionen emittierten Strahlenbündeln - erfaßt wird, kann mit dem eingangs erwähnten Rekonstruktionsalgorithmus von Feldkamp rekonstruiert werden - oder einem anderen Rekonstruktionsalgorithmus für kreisförmige Trajektorien. Wegen der ungewöhnlichen Form dieses Teilvolumens wird man sich jedoch auf den gepunktet angedeuteten, in Fig. 2A mit V₀ bezeichneten ebenen Bereich beschränken. Man erkennt, daß der Bereich V₀ klein ist im Vergleich zu dem von Strahlung durchsetzten Teil des Untersuchungsbereichs.

Außerdem sind in Fig. 2A zwei Seitenflächen dargestellt, die zur Rotationsachse senkrecht sind und die Spitze des Teilvolumens V₁ schneiden. Ein Voxel auf einer der Seitenflächen und am Rand des Untersuchungsbereichs 13 ist mit P₂ bezeichnet.

Fig. 2B zeigt - gegenüber Fig. 2A um 90° gedreht - die kreisförmige Bahn, auf der die Strahlenquelle die Rotationsachse 14 umkreist, sowie das Voxel P₂ auf der Seitenfläche. Der Teil der Kreisbahn, von dem aus P₂ keine Strahlung empfängt, weil die Öffnung des Strahlenbündels in Richtung der Rotationsachse nicht genügend groß ist, ist dabei dünner gezeichnet als der Teil der Bahn, von dem aus das Voxel P₂ Strahlung empfängt. Die Übergänge zwischen den beiden Kreisbögen sind durch die Strahlenquellenpositionen S₁ und S₂ bezeichnet. Man erkennt, daß der dicke Kreisbogen symmetrisch zu einer Winkelposition ϕₛ verläuft, in der ein Strahl von der Strahlenquelle zum Voxel P₂ genau durch die Rotationsachse 14 geht.

Man erkennt aus Fig. 2B weiterhin, daß die Strahlenquelle von der Position S₁ in die Position S₂ um einen Winkel Δβ von mehr als 180° rotieren muß, obwohl der Bestrahlungswinkelbereich Δϕ (das ist der Winkelbereich, den die Parallel-Projektion der Strahlen von der Strahlenquelle zu dem Voxel P₂ auf die x-y-Ebene in dieser Ebene bedeckt) exakt 180° entspricht. Diese Differenz zwischen Δβ und Δϕ ist für Voxel, die näher an der Rotationsachse 14 liegen, geringer. Es läßt sich zeigen, daß alle anderen Voxel auf den Seitenflächen (außerhalb von V₁), die nicht am äußeren Rand des Untersuchungsbereiches liegen, einen Bestrahlungswinkelbereich Δϕ von mehr als 180°, aber weniger als 360° aufweisen.

Für die zwischen den Seitenflächen und den Außenflächen von V₁ liegenden Voxel ist der Bestrahlungswinkelbereich ebenfalls größer als 180° und kleiner als 360°. Da man die Absorption von Voxeln mit einem Bestrahlungswinkelbereich von mindestens 180° rekonstruieren kann (bei CT-Verfahren mit einem ebenen, fächerförmigen Strahlenbündel ist es ja auch möglich, Rekonstruktionsalgorithmen zu benutzen, die mit einem Rekonstruktionswinkelbereich von nur 180° arbeiten), wird dieser Teil des Untersuchungsbereiches (inklusive seiner Seitenflächen) als zweites Teilvolumen V₂ definiert.

Fig. 2C zeigt die Strahlenquellenposition S₁ und S₂ in einer zu den Fig. 2A und Fig. 2B um 90° gedrehten Darstellung. Es sind die beiden Strahlen von den Strahlenquellenpositionen S₁ und S₂ zum Voxel P₂ sowie die Verbindungsgerade zwischen diesen Voxeln dargestellt. Zwischen den beiden Verbindungsgeraden und dem Voxel besteht ein Winkel, der dem Öffnungswinkel des kegelförmigen Strahlenbündels entspricht. In der Darstellung von Fig. 2B, in der die beiden Strahlen auf die x,y-Ebene projiziert werden, wirkt dies so, als seien sie um exakt 180° versetzt.

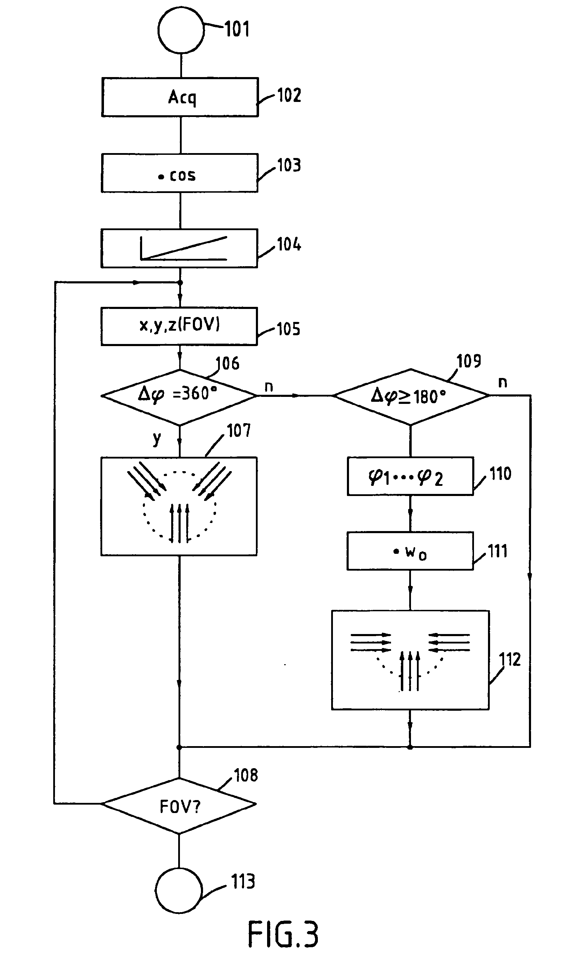

Fig. 3 erläutert den Ablauf eines Rekonstruktionsverfahrens mit dem man die Absorptionsverteilung in einem erheblich größeren Teil des Untersuchungsbereichs rekonstruieren kann.

Nach der Initialisierung im Block 101 rotiert die Gantry mit einer konstanten Winkelgeschwindigkeit. Es wird dann im Schritt 102 die Röntgenstrahlung eingeschaltet, und die dabei von der Detektoreinheit 16 erfaßten Meßdaten werden in einem Speicher des Bildverarbeitungsrechners gespeichert.

Im Verarbeitungsschritt 103 wird jeder Meßwert mit einem Faktor gewichtet (multipliziert), der proportional ist zu dem Kosinus des Winkels, den der Strahl, zu dem der jeweilige Meßwert gehört, mit dem Zentralstrahl einschließt (der Zentralstrahl ist der Strahl, der von der Strahlenquelle S ausgehend die Rotationsachse 14 senkrecht schneidet und auf die Mitte der Detektoreinheit 16 trifft).

Im Schritt 104 werden die von einer Detektorzeile (die sich in der x,y-Ebene befindet) gelieferten und gemäß dem Schritt 103 gewichteten Meßwerte einer Hochpaßfilterung unterzogen. Wenn die Detektoreinheit 16 eben ist, ist diese Filterung rampenförmig, d.h. sie hat einen linear mit der Ortsfrequenz zunehmenden Übertragungsfaktor. Wenn die Detektoreinheit bogenförmig um die Strahlenquelle S oder um die Rotationsachse 14 gekrümmt ist, muß diese Filterung bekanntlich modifiziert werden.

Nachdem alle Meßdaten auf diese Weise gemäß den Schritten 103 und 104 verarbeitet worden sind, wird ein Voxel x,y,z innerhalb eines vorgebbaren Bereiches (field of view - FOV) vorgegeben (Schritt 105). Im Schritt 106 verzweigt das Ablaufdiagramm, je nachdem, ob der Bestrahlungswinkelswinkelbereich Δϕ für dieses Voxel kleiner ist als 360° oder nicht. Bei einer vorgegebenen Geometrie des Computertomographen kann in einer look up-Tabelle für jedes Voxel in einem mit der Gantry verbundenen Koordinatensystem gespeichert sein, ob die Bedingung Δϕ = 360° erfüllt ist oder nicht.

Wenn die Bedingung erfüllt ist, d.h. wenn sich das betreffende Voxel während des gesamten Umlaufs im Strahlenbündel befunden hat, erfolgt im Schritt 107 eine Rückprojektion der gefilterten Daten, wobei für das betreffende Voxel die Meßdaten von allen Strahlen herangezogen werden, die während der Akquisition der Meßdaten dieses Voxel passiert haben. Jeder Meßwert wird dabei mit einem sogenannten "Vergrößerungsfaktor" (magnification factor) multipliziert, der von dem Abstand zwischen diesem Voxel und der jeweiligen Strahlenquellenposition abhängt, in der der Meßwert akquiriert wurde.

Wenn in einer Strahlenquellenposition kein Strahl exakt durch das betreffende Voxel verläuft, kann für diese Strahlenquellenposition ein Strahl (bzw. ein gefilterter Meßwert) durch Interpolation der Meßwerte von mehreren Strahlen gefunden werden.

Nachdem auf diese Weise für alle Strahlenquellenpositionen die Beiträge zu dem betreffenden Voxel akkumuliert worden sind, wird im Schritt 108 geprüft, ob alle Voxel in dem zu rekonstruierenden Bereich FOV durchlaufen sind. Ist dies nicht der Fall, verzweigt das Ablaufdiagramm zum Schritt 105.

Die Folge der Schritte 103 ...107 entspricht im wesentlichen dem von Feldkamp angegebenen Rekonstruktionsalgorithmus. Allerdings kann damit die Absorption nur für die Voxel rekonstruiert werden, die in allen Strahlenquellenpositionen von Strahlung getroffen wurden, bzw. die in den zur Rotationsachse 14 rotationssymmetrischen, diskusförmigen Bereich V₁ liegen. Das hybride Rekonstruktionsverfahren gemäß der Erfindung gestattet demgegenüber die Rekonstruktion der Absorptionsverteilung in einem größeren Bereich.

Wenn feststeht, daß das Voxel x,y,z nicht im Teilvolumen V₁ enthalten ist, d.h. nicht in allen Strahlenquellenpositionen von Strahlung getroffen wird, erfolgt im Schritt 109 eine Abfrage, ob sich für das vorgegebene Voxel ein Bestrahlungswinkelbereich Δϕ von wenigstens 180° ergibt (auch dies kann in einer look-up Tabelle gespeichert sein). Ist dies nicht der Fall, dann ist die Absorption in dem betreffenden Voxel nicht exakt rekonstruierbar und das Programm verzweigt zur Abfrage 108. Wenn hingegen der Bestrahlungsbereich Δϕ mindestens 180° beträgt, wird die Absorption auch in diesem Voxel mit dem im folgenden erläuterten Rekonstruktionsalgorithmus ermittelt.

Dabei wird zunächst im Schritt 110 der Bestrahlungswinkelbereich Δϕ festgelegt, der zur Rekonstruktion der Absorption in dem Voxel herangezogen wird. Es sei angenommen, daß das vorgegebene Voxel x,y,z auf den äußeren Seitenflächen des Teilvolumens V₂ und am Rand des Untersuchungsbereiches 13 liegt - wie etwa das Voxel P₂. In diesem Fall bleibt keine andere Wahl, als den gesamten als den gesamten Bestrahlungswinkelbereich Δϕ zwischen den Strahlenquellenpositionen S₁ zur Rekonstruktion heranzuziehen, weil dieser die Bedingung Δϕ =180° gerade erfüllt.

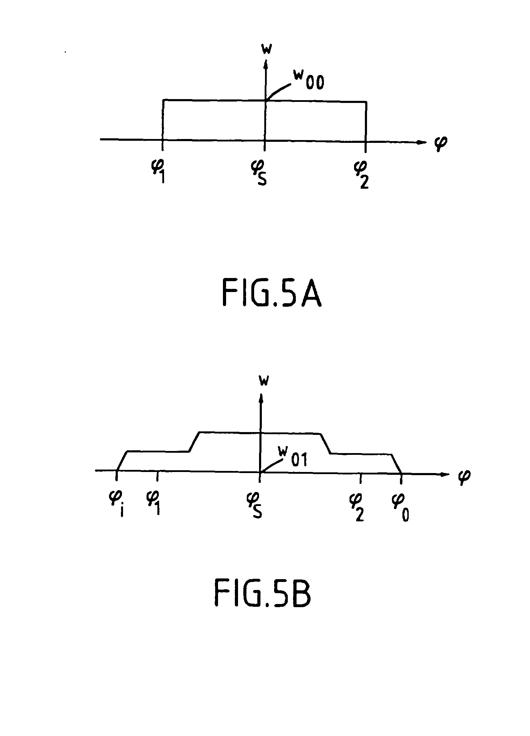

Die Meßwerte aller Strahlen im Bestrahlungswinkelbereich Δϕ durch dieses Voxel werden im Schritt 111 mit einem Gewichtungsfaktor w₀ gewichtet. Gemäß Fig. 5a, die die Abhängigkeit dieses Gewichtungsfaktors w vom Bestrahlungswinkel ϕ darstellt, ist w₀ von ϕ unabhängig ; ϕ₁ und ϕ₂ sind dabei die zu den Strahlenquellenpositionen S₁ und S₂ korrespondierenden Bestrahlungswinkel (in einer Projektion auf die x,y-Ebene). Der Gewichtungsfaktor w₀ ist dem Wert 1/N proportional, wobei N die Zahl der Strahlenquellenpositionen auf dem Kreisbogen zwischen S₁ und S₂ (und damit die Zahl der Strahlen, die durch das betreffende Voxel verlaufen) ist. Dadurch werden die weiteren Schritte unabhängig von dem Wert N bzw. von dem Winkel Δβ, den die Strahlenquelle auf dem Kreisbogen von S₁ bis S₂ durchläuft. Wie zuvor ausgeführt, hängen der Winkel Δβ bzw. die Zahl N von dem Abstand des jeweiligen Voxels von der Rotationsachse 14 ab.

Im Schritt 112 erfolgt dann eine Rückprojektion, wobei die Meßwerte der über den Bestrahlungswinkelbereich von 180° verteilten Strahlen mit dem Vergrößerungsfaktor multipliziert werden, der von dem Abstand zwischen der zu diesem Strahl gehörenden Strahlenquellenposition und dem Voxel abhängig ist. Die vom Bestrahlungswinkel ϕ unabhängige Gewichtung und die Multiplikation mit dem vom Bestrahlungswinkel ϕ abhängigen Vergrößerungsfaktor, können auch in einem einzigen Schritt durchgeführt werden.

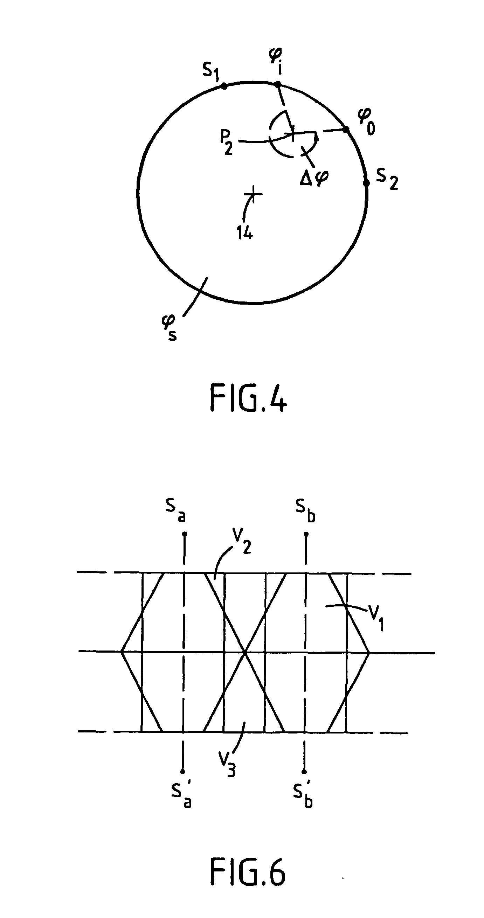

Wenn das zu rekonstruierende Voxel innerhalb des Teilvolumens V₂ (aber außerhalb des Teilvolumens V₁) liegt, wie z.B. das Voxel P'₂ in Fig. 2A, sind die Verhältnisse etwas anders als bei einem Voxel auf der Seitenfläche und am Rand des Untersuchungsbereiches 13 (wie etwa P₂). Dies ist anhand von Fig. 4 erläutert, die in einer zu Fig. 2B analogen Darstellung die Verhältnisse für das Voxel P'₂ (vgl. Fig. 2A) zeigt. Es ist wiederum die Winkelposition ϕₛ dargestellt, und mit S₁ und S₂ sind die dazu symmetrischen Strahlenquellenpostionen dargestellt, aus denen die Strahlen zum Voxel P'₂ (genauer: deren Projektionen auf die x,y-Ebene) um genau 180° gegeneinander versetzt sind. Fig. 4 zeigt, daß es jenseits dieser Strahlenquellenpostionen noch weitere Positionen in dem dick ausgezogenen Bereich gibt, aus denen das Voxel P'₂ bestrahlt wird. In diesem Fall gibt es für die Schritte 110 und 111 folgende Möglichkeiten:
a) aus dem gesamten zur Verfügung stehenden Bestrahlungswinkelbereich ϕᵢ - ϕ₀ wird ein Bereich herausgeschnitten, der genau 180° entspricht, z.B. ein zu der Symmetrieposition ϕₛ symmetrischer Bereich, der durch die Strahlenquellenposition S₁ und S₂ bzw. (vgl. Fig. 5a) durch ϕ₁ und ϕ₂ gekennzeichnet ist. Der herausgeschnittene Bereich muß aber nicht symmetrisch zu ϕₛsein. Der Gewichtungsfaktor w₀₀ mit dem alle Meßwerte in gleicher Weise gewichtet werden, ist dabei wiederum dem Wert 1/N proportional.
b) eine zweite Möglichkeit besteht darin, auch die Strahlen mit einem Bestrahlungswinkel außerhalb des Bereichs ϕ₁ und ϕ₂ heranzuziehen und diese Strahlen und einen entsprechenden Teil der innerhalb von ϕ₁ und ϕ₂ befindlichen Strahlrichtungen so zu gewichten, daß die Strahlen durch das Voxel P'₂, deren Projektionen in die x,y-Ebene in einander exakt entgegengesetzte Richtungen verlaufen, zusammen das gleiche Gewicht erhalten, wie z.B. der durch den Strahlungswinkel ϕₛ gekennzeichnete Einzelstrahl, zu dem es kein (um 180° versetztes) Gegenstück gibt. Da die äußeren Strahlen dabei also mit einem geringeren Gewicht eingehen als die Strahlen in der Mitte, ist diese Rekonstruktion einer Rekonstruktion mit einem Rekonstruktionswinkelbereich von 180° (Fig. 5a) äquivalent.

Nachdem auf diese Weise die Absorption für alle Voxel, die in dem FOV sowie in den Teilvolumina V₁ und V₂ liegen, ermittelt ist, ist das hybride Rekonstruktionsverfahren beendet.

Anstelle des Feldkamp-Algorithmus kann auch ein anderer Rekonstruktionsalgorithmus zur Rekonstruktion der Absorption der Voxel in dem Teilvolumen V₁ verwendet werden. Ebenso kann die Absorptionsverteilung in dem Teilvolumen V₂ mit einem anderen Rekonstruktionsalgorithmus rekonstruiert werden, der die Rekonstruktion aus einem Bestrahlungswinkelbereich von weniger als 360° gestattet.

Wie die Fig. 2A zeigt, ist die durch die Teilvolumina V₁+V₂ definierte Scheibe, innerhalb der die Rekonstruktionsverteilung rekonstruiert werden kann, deutlich breiter als die Scheibe V₀, in der die Absorptionsverteilung rekonstruiert werden kann, wenn man sich auf den Feldkamp-Algorithmus allein beschränkt. Wenn ein FOV mit einem kleineren Durchmesser als der Untersuchungsbereich 13 vorgegeben wird, kann die Scheibe V₁+V₂ sogar noch dicker sein. Es läßt nämlich zeigen, daß alle Punkte innerhalb des Untersuchungsbereiches mit einem Bestrahlungswinkelbereich Δϕ = 180° auf einer konvexen und bezüglich der Rotationsachse 14 rotationssymmetrischen Fläche F liegen. Diese in Fig 2a gestrichelt angedeutete Flächen F tangieren die Spitzen des Konus. Jede Scheibe innerhalb der Flächen, aber auch das durch die Flächen F begrenzte Volumen selbst, läßt sich vollständig rekonstruieren. Dabei werden die Voxel zwischen den Flächen F und dem durch das Teilvolumen V₁ definierten Kegel dem Teilvolumen V₂ und die Voxel innerhalb des Kegels dem Teilvolumen V₁ zugeordnet.

Trotzdem kann aber auch das durch die Flächen F definierte Gesamtvolumen für manchen Anwendungsfall nicht ausreichend sein. In diesem Fall kann der Untersuchungsbereich auf zwei in Richtung der Rotationsachse gegeneinander versetzten Kreisen abgetastet werden. Dies ist in Fig. 6 dargestellt, wobei mit Sₐ und S'ₐ zwei um 180° auf dem einen Kreis und mit S_{b} und S'_{b} zwei auf dem anderen Kreis um 180° versetzte Strahlenquellenpositionen angedeutet sind. Die Detektoreinheiten sind aus Gründen der Übersichtlichkeit nicht dargestellt, jedoch die resultierenden Teilvolumen V₁ und V₂. Die Rekonstruktion für die Teilvolumina V₁ und V₂ kann wiederum so erfolgen wie in Verbindung mit Fig. 3 beschrieben.

In der Mitte zwischen den Kreisbahnen befindet sich ein ebenes, scheibenförmiges Teilvolumen V₃, das beiderseits durch die zu dem Teilvolumen V₂ gehörenden Seitenflächen (Fig. 2A) begrenzt wird. Zumindest die am äußeren Rand des Untersuchungsbereiches in dem Teilvolumen befindlichen Voxel haben einen Bestrahlungswinkelbereich von weniger als 180°.

Die Zuordnung zu den Teilvolumina V₂ oder V₁ erfolgt dabei wiederum in Abhängigkeit vom Bestrahlungswinkelbereich Δϕ. Die Zuordnung zu dem Teilvolumen V₃ hingegen erfolgt in Abhängigkeit von der Lage, d.h. von der z-Koordinate eines Voxels.

Die Rekonstruktion der Absorptionsverteilung in dem Volumen V₃ erfolgt zweckmäßigerweise mit einem ART-Verfahren. ART-Verfahren sind iterative Verfahren, bei denen zunächst den zu rekonstruierenden Voxeln ein geeigneter Absorptionswert zugeordnet wird, wonach die Absorptionswerte von auf demselben Strahl liegenden Voxeln akkumuliert werden und mit dem für diesen Strahl akquirierten Meßwert verglichen werden. Die Differenz wird auf geeignete Weise auf die auf diesem Strahl liegenden Voxel verteilt. Nachdem auf diese Weise die Absorptionsverteilung in allen Voxeln des zu rekonstruierenden Volumens korrigiert worden ist, wird der beschriebene Vergleich mit den akquirierten Meßdaten wiederholt usw. Das ART-Verfahren gestattet näherungsweise die Rekonstruktion der Absorption auch in den Voxeln mit einem Bestrahlungswinkelbereich Δϕ < 180°.

Die Erfindung ist nicht nur bei solchen CT-Verfahren anwendbar, bei denen eine kreisförmige Relativbewegung stattfindet, sondern auch bei solchen CT-Verfahren, bei denen in Folge eines Vorschubs in z-Richtung eine helixförmige Relativbewegung zwischen Untersuchungsbereich erfolgt und Strahlenquelle bzw. Detektor erfolgt.

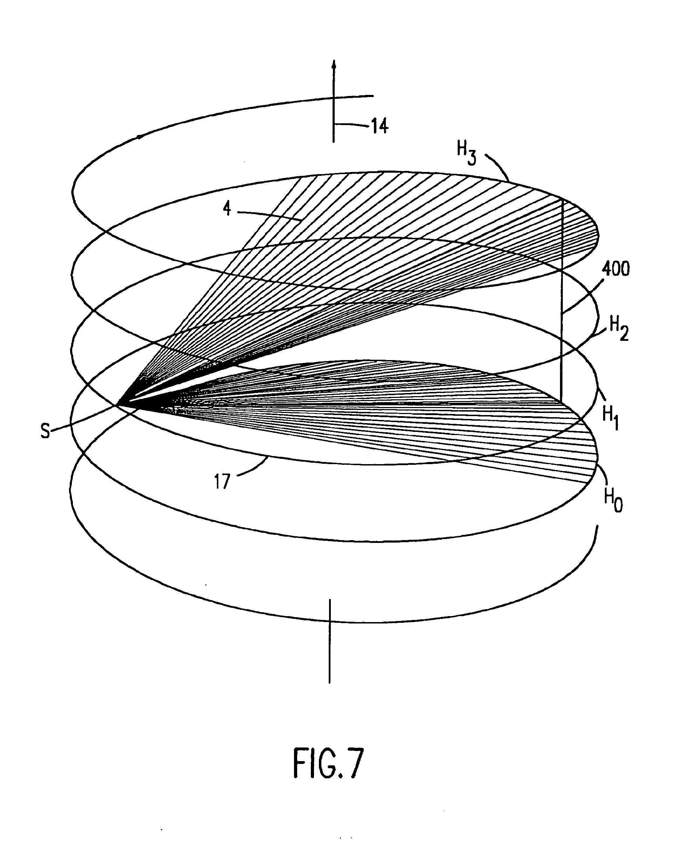

Im Prinzip ist es bei einer helixförmigen Abtastbewegung gleichgültig, ob die Strahlenquelle S und die Detektoreinheit 16 oder der Untersuchungsbereich (bzw. das daran befindliche Objekt) die Rotations- bzw. Vorschubbewegung ausführen, wesentlich ist allein die Relativbewegung. Deshalb ist in Fig. 7 angenommen, daß sich die Strahlenquelle S (und die mit ihr über die Gantry 1 verbundene, in Fig.7 nicht dargestellte Detektoreinheit 16) auf der helixförmigen Bahn 17 nach oben bewegen, während der Untersuchungsbereich 13, ebenso wie das darin befindliche Objekt - in Fig. 7 nicht dargestellt ist - ruht.

Bei einem in der Offenlegunsschrift DE 19 835 296 A1 beschriebenen Verfahren dieser Art sind die Abmessungen der Detektoreinheit, das von der Strahlenquelle 4 emittierte Strahlenbündel, die Vorschubgeschwindigkeit und die Rotationsgeschwindigkeit so aufeinander abgestimmt, daß von der Detektoreinheit gerade die Strahlen erfaßt werden, die mit den Windungen H₀ und H₃ der Helix 17 zusammenfallen. Weil sich dazwischen noch die beiden Helixwindungen H₁ und H₂ befinden, haben diese voneinander einen Abstand von dem dreifachen (allgemein dem (2n+1) - fachen) des Abstandes zweier benachbarter Helixwindung.

Es läßt sich zeigen, daß in diesem Fall alle Voxel, die nach dem Beginn der Abtastung des Untersuchungsbereichs in das Strahlenbündel eintreten und es vor dem Ende der Abtastung verlassen, von der Strahlenquelle unter einem Winkel von exakt 3π (allgemein (2n+1)π) bestrahlt worden sind, wodurch eine sehr einfache Rekonstruktion mit sehr guter Bildqualität möglich wird.

Für die Voxel, die sich bei Beginn der Abtastbewegung schon in dem Strahlenbündel 4 befinden, und die Voxel, die sich am Ende der Abtastbewegung noch darin befinden, gilt dies nicht. Für sie ist der Bestrahlungswinkelbereich kleiner, so daß sie sich mit dem in dem genannten Dokument beschriebenen Verfahren nicht rekonstruieren lassen.

Auch hier ist jedoch wiederum eine hybride Rekonstruktion möglich, indem man alle Voxel, die sich am Beginn und am Ende der helixförmigen Relativbewegung außerhalb des Strahlenbündels befinden, einem ersten Teilvolumen zuordnet, dessen Absorptionsverteilung man mit dem in dem genannten Dokument beschriebenen Rekonstruktionsalgorithmus rekonstruiert. Ein Teil der zu Beginn der Bestrahlung im Strahlengang befindlichen Voxel, nämlich die Voxel, die von der Strahlenquelle zwischen die Windungen H₁ und H₂ projiziert werden, wird aus einem Winkelbereich von mehr als 180° bestrahlt. Dieser kann daher rekonstruiert werden, wenn man die Voxel in diesem Teil einem zweiten Teilvolumen zuordnet und die Absorptionsverteilung darin mit einem aus der WO 98/30980 bekannten Verfahren rekonstruiert, wobei man zur Rekonstruktion jeweils nur die Strahlen für einen Winkelbereich von exakt 180° heranzieht.

Die Voxel, die zu Beginn der Bestrahlung auf den Bereich zwischen den Helixwindungen H₀ und H₁ projiziert werden, werden aus einem Bereich von mehr als 2π (aber weniger als 3π) bestrahlt. Diese kann man einem dritten Teilvolumen zuordnen, dessen Absorptionsverteilung man rekonstruiert in dem man für jedes Voxel darin Strahlen aus einem Abtastwinkelbereich von 360° heranzieht.

## Patentansprüche

1. Computertomographie-Verfahren mit den Schritten
- Erzeugen eines kegelförmigen, einen Untersuchungsbereich (13) bzw. ein darin befindliches Objekt durchsetzenden Strahlenbündels (4),
- Erzeugung einer eine Rotation um eine Rotationsachse (14) umfassenden Relativbewegung zwischen dem Strahlenbündel und dem Untersuchungsbereich bzw. dem Objekt
- Akquisition von Meßdaten, die von der Intensität in dem Strahlenbündel jenseits des Untersuchungsbereiches abhängen während der Relativbewegung,
- Rekonstruktion der räumlichen Verteilung der Absorption innerhalb des Untersuchungsbereiches (13) aus den von der Detektoreinheit (16) akquirierten Meßdaten **gekennzeichnet durch** folgende Schritte zur Rekonstruktion der räumlichen Verteilung der Absorption:
a) Definition von mindestens einem ersten und einem zweiten Teilvolumen (V₁; V₂) innerhalb des vom Strahlenbündel durchsetzten Gesamtvolumens, wobei in dem ersten Teilvolumen Randbedingungen eines ersten Rekonstruktionsalgorithmus erfüllt sind und wobei in dem zweiten Teilvolumen Randbedingungen eines zweiten Rekonstruktionsalgorithmus erfüllt sind, wobei der erste Rekonstruktionsalgorithmus einen Rekonstruktionswinkelbereich von 360° ausnutzt, und wobei der zweite Rekonstruktionsalgorithmus mit einem Rekonstruktionswinkelbereich von 180° auskommt,
b) Rekonstruktion der räumlichen Verteilung der Absorption innerhalb des ersten Teilvolumens mittels des ersten Rekonstruktionsalgorithmus,
c) Rekonstruktion der räumlichen Verteilung der Absorption innerhalb des zweiten Teilvolumens mittels des zweiten, vom ersten abweichenden Rekonstruktionsalgorithmus.

2. Computertomographie -Verfahren nach Anspruch 1, wobei die Relativbewegung die Form eines Kreises um die Rotationsachse hat,
**gekennzeichnet durch** folgende Schritte:
a) Zuordnung von Voxeln mit einem Bestrahlungswinkelbereich von 360° zu dem ersten Teilvolumen,
b) Zuordnung von Voxeln mit einem Bestrahlungswinkelbereich von mindestens 180° aber weniger als 360° zu dem zweiten Teilvolumen,
c) Rekonstruktion der Absorption der Voxel in dem ersten Teilvolumen mit einem Rekonstruktionswinkelbereich von 360°,
d) Rekonstruktion der Absorption der Voxel entsprechend einem Rekonstruktionswinkelbereich von 180° in dem zweiten Teilvolumen.

3. Computertomographie-Verfahren nach Anspruch 2,
**gekennzeichnet durch** folgende Schritte:
a) Definition der um 180° gegeneinander versetzten Randstrahlen des Rekonstruktionswinkelbereichs für jedes Voxel im zweiten Teilvolumen
b) Nichtberücksichtigung der Beiträge der außerhalb des so definierten Rekonstruktionswinkelbereichs **durch** das betreffende Voxel verlaufenden Strahlen.

4. Computertomographie-Verfahren nach Anspruch 2,
**gekennzeichnet durch** folgende Schritte:
a) Gewichtung der Beiträge von Strahlen-Paaren, die mit um 180° gegeneinander versetzten Richtungen **durch** ein Voxel im zweiten Teilvolumen verlaufen, derart dass ihr Gesamtgewicht so groß ist wie das eines Einzelstrahls
b) Summierung aller - ggf. gewichteten - Beiträge von Strahlen, die **durch** das betreffende Voxel verlaufen
c) Wiederholung der Schritte a) und c) für andere Voxel im zweiten Teilvolumen.

5. Computertomographie-Verfahren nach Anspruch 1, wobei der Untersuchungsbereich auf zwei gegeneinander in Richtung der Rotationsachse versetzten Kreisen abgetastet wird,
**gekennzeichnet durch** folgende Schritte:
a) Zuordnung von Voxeln außerhalb eines ebenen, die Rotationsachse senkrecht schneidenden Zwischenbereiches mit einem Bestrahlungswinkelbereich von 360° zu einem ersten Teilvolumen,
b) Zuordnung von außerhalb des Zwischenbereiches befindlichen Voxeln mit einem Bestrahlungswinkelbereich von mindestens 180°, aber weniger als 360° zu einem zweiten Teilvolumen,
c) Zuordnung von Voxeln innerhalb des Zwischenbereiches mit einem zu einem dritten Teilvolumen,
d) Rekonstruktion der Voxel innerhalb des dritten Teilvolumens mit einem ART-Verfahren.

6. Computertomographie-Verfahren nach Anspruch 1, wobei die Relativbewegung in Form einer Helix (17) eine Rotation um eine Rotationsachse (14) und einen Vorschub in Richtung parallel zur Rotationsachse umfasst und wobei sich für Voxel im Untersuchungsbereich ein Bestrahlungswinkelbereich von genau (2n + 1)π ergibt, **gekennzeichnet durch** folgende Schritte:
a) Zuordnung von vom Strahlenbündel erfassten Voxeln, die sich am Beginn und am Ende der helixförmigen Relativbewegung außerhalb des kegelförmigen Strahlenbündels befinden, zu einem ersten Teilvolumen,
b) Zuordnung von Voxeln, die sich am Beginn oder am Ende der helixförmigen Relativbewegung innerhalb des Strahlenbündels befinden, zu dem zweiten Teilvolumen,
c) Rekonstruktion der Absorptionsverteilung in den beiden Teilvolumina mit unterschiedlichen Rekonstruktionsalgorithmen.

7. Computertomograph zur Durchführung des Verfahrens nach Anspruch 1 mit
- einer Strahlenquelle (S) zur Emission eines kegelförmigen, einen Untersuchungsbereich (13) bzw. ein darin befindliches Objekt durchsetzenden Strahlenbündels,
- eine mit der Strahlenquelle verbundene zweidimensionale Detektoreinheit (16) zur Akquisition von der Intensität in dem Strahlenbündel jenseits des Untersuchungsbereiches abhängigen Meßdaten,
- einer Antriebsanordnung (2,5) zur Erzeugung einer eine Rotation um eine Rotationsachse (14) umfassenden Relativbewegung zwischen der Strahlenquelle (S) und der Detektoreinheit (16) einerseits und dem Untersuchungsbereich (13) bzw. dem Objekt andrerseits,
- und mit einer Rekonstruktionseinheit (10) zur Rekonstruktion der räumlichen Verteilung der Absorption innerhalb des Untersuchungsbereiches (13) aus den von der Detektoreinheit (16) akquirierten Meßdaten
**gekennzeichnet dadurch, dass** der Computertomograph für folgende Schritte zur Rekonstruktion der räumlichen Verteilung der Absorption ausgebildet ist:
a) Definition von mindestens einem ersten und einem zweiten Teilvolumen innerhalb des vom Strahlenbündel durchsetzten Gesamtvolumens, wobei in dem ersten Teilvolumen Randbedingungen eines ersten Rekonstruktionsalgorithmus erfüllt sind und wobei in dem zweiten Teilvolumen Randbedingungen eines zweiten Rekonstruktionsalgorithmus erfüllt sind, wobei der erste Rekonstruktionsalgorithmus einen Rekonstruktionswinkelbereich von 360° ausnutzt, und wobei der zweite Rekonstruktionsalgorithmus mit einem Rekonstruktionswinkelbereich von 180° auskommt,
b) Rekonstruktion der räumlichen Verteilung der Absorption innerhalb des ersten Teilvolumens mittels des ersten Rekonstruktionsalgorithmus
c) Rekonstruktion der räumlichen Verteilung der Absorption innerhalb des zweiten Teilvolumens mittels des zweiten, vom ersten abweichenden Rekonstruktionsalgorithmus.

## Claims

1. A computer tomography process comprising the following steps:
- Generation of a cone-shaped radiation beam (4) passing through an examination region (13) or an object present therein;
- Generation of a rotation about a rotational axis (14) with relative movement between the radiation beam and the examination region or the object;
- Acquisition of measurement data, which depend on the intensity of the radiation beam beyond the examination region during the relative movement;
- Reconstruction of the spatial absorption distribution within the examination region (13) from the measurement data acquired by the detector unit (16), **characterized by** the following steps for reconstruction of the spatial absorption distribution.
a) Definition of at least a first and a second partial volume (V1, V2) within the total volume traversed by the radiation beam, whereby in the first partial volume the boundary conditions of a second reconstruction algorithm are satisfied, whereby the first reconstruction algorithm uses a reconstruction angle range of 360°, and whereby the second reconstruction algorithm manages with a reconstruction angle range of 180°.
b) Reconstruction of the spatial absorption distribution within the first partial volume by means of the first reconstruction algorithm.
c) Reconstruction of the spatial absorption distribution within the second partial volume by means of the second reconstruction algorithm, deviating from the first.

2. The computer tomography process of claim 1, whereby the relative movement has the form of a circle around the rotational axis, **characterized by** the following steps:
a) Assignment of voxels with an irradiation angle range of 360° to the first partial volume.
b) Assignment of voxels with an irradiation angle range of at least 180° but less than 360° to the second partial volume.
c) Reconstruction of the absorption of the voxels in the first partial volume with a reconstruction angle range of 360°.
d) Reconstruction of the absorption of the voxels corresponding to a reconstruction angle range of 180° in the second partial volume.

3. The computer tomography process according to claim 2, **characterized by** the following steps:
a) Definition of the 180° offset reconstruction edge radiation beams of the reconstruction angle range for each voxel in the second partial volume.
b) Non-consideration of the contribution from radiation beams passing through the relevant voxel outside of the thus defined reconstruction angle range.

4. The computer tomography process according to claim 2, **characterized by** the following steps:
a) Weighting of the contribution of radiation beam pairs that pass through a voxel in the second partial volume, with directions offset 180°, so that their total weighting is as large as that of a single radiation beam.
b) Summation of all - possibly weighted - contributions of radiation beams passing through the relevant voxel.
c) Repetition of steps a) and c) for other voxels in the second partial volume.

5. The computer tomography process according to claim 1, whereby the examination region is read on two circles offset against each other in the direction of the rotational axis, **characterized by** the following steps:
a) Assignment to a first partial volume of voxels outside a plane of the intermediate region perpendicularly intersecting the rotation axis with an irradiation angle range of 360°.
b) Assignment to the second partial volume of voxels outside the intermediate region with an irradiation angle range of at least 180° but less than 360°.
c) Assignment to a third partial volume of voxels within the intermediate region.
d) Reconstruction of the voxels within the third partial volume using an ART process.

6. The computer tomography process according to claim 1, whereby the relative movement in the form of a helix (17) comprises a rotation about a rotational axis (14) and an advance in a direction parallel to the rotational axis, and whereby there is an irradiation angle range of exactly (2n +1) π for voxels in the examination region, **characterized by** the following steps:
a) Assignment to a first partial volume of the voxels read by the radiation beam located at the beginning and at the end of the helical relative movement outside of the cone-shaped radiation beam.
b) Allocation to the second partial volume of the voxels located at the beginning or the end of the helical relative movement within the radiation beam.
c) Reconstruction of the absorption distribution in both partial volumes with different reconstruction algorithms.

7. The computer tomograph for carrying out the process according to claim 1, comprising a radiation source (S) for emitting a cone-shaped radiation beam traversing an examination region (13) or an object present therein,
- a two-dimensional detector unit (16) connected to the radiation source for the acquisition of the intensity of the radiation beam beyond the examination region-dependent measurement data;
- a drive device (2, 5) for generating a rotation about a rotational axis (14) with relative movement between the radiation source (S) and the detector unit (16), on the one hand, and the examination region (13) or the object, on the other;
- and, with a reconstruction unit (10) for reconstruction of the spatial absorption distribution within the examination region (13) from the measurement data acquired by the detector unit (16), **characterized in that** the computer tomograph is designed to carry out the following steps for reconstruction of the spatial absorption distribution.
a) Definition of at least a first and a second partial volume within the total volume traversed by the radiation beam, whereby in the first partial volume the boundary conditions of a first reconstruction algorithm are satisfied and whereby in the second partial volume the boundary conditions of a second reconstruction algorithm are satisfied, whereby the first reconstruction algorithm uses a reconstruction angle range of 360°, and whereby the second reconstruction algorithm manages with a reconstruction angle range of 180°.
b) Reconstruction of the spatial absorption distribution within the first partial volume by means of the first reconstruction algorithm.
c) Reconstruction of the spatial absorption distribution within the second partial volume by means of the second reconstruction algorithm, deviating from the first.

## Revendications

1. Procédé de tomodensitométrie comprenant les étapes de
- génération d'un faisceau de rayons coniques (4) traversant une zone d'analyse (13) ou un objet s'y trouvant,
- génération d'un mouvement relatif comprenant une rotation autour d'un axe de rotation (14) entre le faisceau de rayons et la zone d'analyse ou l'objet,
- acquisition de données de mesure qui dépendent de l'intensité dans le faisceau de rayons au-delà de la zone d'analyse pendant le mouvement relatif,
- reconstruction de la répartition spatiale de l'absorption dans la zone d'analyse (13) à partir des données de mesure acquises par l'unité de détecteur (16), **caractérisé par** les étapes suivantes de reconstruction de la répartition spatiale de l'absorption :
a) définition d'au moins un premier et un deuxième volumes partiels (V₁ ; V₂) au sein du volume total traversé par le faisceau de rayons, des conditions aux limites d'un premier algorithme de reconstruction étant satisfaites dans le premier volume partiel et des conditions aux limites d'un deuxième algorithme de reconstruction étant satisfaites dans le deuxième volume partiel, le premier algorithme de reconstruction utilisant un angle de reconstruction de 360° et le deuxième algorithme de reconstruction présentant un angle de reconstruction de 180°,
b) reconstruction de la répartition spatiale de l'absorption dans le premier volume partiel au moyen du premier algorithme de reconstruction,
c) reconstruction de la répartition spatiale de l'absorption dans le deuxième volume partiel au moyen du deuxième algorithme de reconstruction divergeant du premier.

2. Procédé de tomodensitométrie selon la revendication 1, dans lequel le mouvement relatif a la forme d'un cercle autour de l'axe de rotation, **caractérisé par** les étapes suivantes :
a) affectation de voxels avec un angle de rayonnement de 360° au premier volume partiel,
b) affectation des voxels avec un angle de rayonnement d'au moins 180° mais inférieur à 360° au deuxième volume partiel,
c) reconstruction de l'absorption des voxels dans le premier volume partiel avec un angle de reconstruction de 360°,
d) reconstruction de l'absorption des voxels correspondant à un angle de reconstruction de 180° dans le deuxième volume partiel.

3. Procédé de tomodensitométrie selon la revendication 2, **caractérisé par** les étapes suivantes :
a) définition de rayons limites de l'angle de reconstruction, déplacés de 180° les uns par rapport aux autres, pour chaque voxel dans le deuxième volume partiel,
b) non prise en compte des contributions des rayons évoluant en dehors de l'angle de reconstruction ainsi défini.

4. Procédé de tomodensitométrie selon la revendication 2, **caractérisé par** les étapes suivantes :
a) obtention des contributions des paires de rayons qui passent dans des directions décalées les unes par rapport aux autres de 180° par un voxel dans le deuxième volume partiel de telle sorte que son poids total soit aussi élevé que celui d'un rayon individuel,
b) addition de toutes les contributions ou des contributions pondérées de rayons qui passent par le voxel concerné,
c) répétition des étapes a) et c) pour d'autres voxels dans le deuxième volume partiel.

5. Procédé de tomodensitométrie selon la revendication 1, dans lequel la zone d'analyse est détectée sur deux cercles décalés l'un par rapport à l'autre dans la direction de l'axe de rotation, **caractérisé par** les étapes suivantes :
a) affectation de voxels en dehors d'une zone intermédiaire coupant perpendiculairement l'axe de rotation avec un angle de rayonnement de 360° à un premier volume partiel,
b) affectation de voxels se trouvant en dehors de la zone intermédiaire avec un angle de rayonnement d'au moins 180° mais inférieur à 360° à un deuxième volume partiel,
c) affectation de voxels dans la deuxième zone intermédiaire avec volume partiel à un troisième volume partiel,
d) reconstruction des voxels dans le troisième volume partiel avec un procédé ART.

6. Procédé de tomodensitométrie selon la revendication 1, dans lequel le mouvement relatif sous la forme d'une hélice (17) comprend une rotation autour d'un axe de rotation (14) et une avancée dans une direction parallèle à l'axe de rotation et dans lequel on a pour les voxels dans la zone d'analyse, un angle de rayonnement d'exactement (2n + 1) π, **caractérisé par** les étapes suivantes :
a) affectation des voxels acquis par le faisceau de rayons qui se trouvent au début et à la fin du mouvement relatif hélicoïdal en dehors du faisceau de rayons coniques, à un premier volume partiel,
b) affectation des voxels qui se trouvent au début ou à la fin du mouvement relatif hélicoïdal dans le faisceau de rayons au deuxième volume partiel,
c) reconstruction de la répartition d'absorption dans les deux volumes partiels avec des algorithmes de reconstruction différents.

7. Tomodensitomètre pour la réalisation du procédé selon la revendication 1, comprenant
- une source de rayons (S) pour l'émission d'un faisceau de rayons coniques traversant une zone d'analyse (13) ou un objet s'y trouvant,
- une unité de détecteur (16) bidimensionnelle reliée à la source de rayons pour l'acquisition de données de mesure dépendant de l'intensité dans le faisceau de rayons au-delà de la zone d'analyse pendant le mouvement relatif,
- un dispositif d'entraînement (2, 5) pour générer un mouvement relatif comprenant une rotation autour d'un axe de rotation (14) entre la source de rayons (S) et l'unité de détecteur (16) d'une part et la zone d'analyse (13) ou l'objet d'autre part,
- et une unité de reconstruction (10) pour la reconstruction de la répartition spatiale de l'absorption dans la zone d'analyse (13) à partir des données de mesure acquises de l'unité de détecteur (16), **caractérisé en ce que** le tomodensitomètre est conçu pour les étapes suivantes pour la reconstruction de la répartition spatiale de l'absorption :
a) définition d'au moins un premier et un deuxième volumes partiels au sein du volume total traversé par le faisceau de rayons, des conditions aux limites d'un premier algorithme de reconstruction étant satisfaites dans le premier volume partiel et des conditions aux limites d'un deuxième algorithme de reconstruction étant satisfaites dans le deuxième volume partiel, le premier algorithme de reconstruction utilisant un angle de reconstruction de 360° et le deuxième algorithme de reconstruction présentant un angle de reconstruction de 180°,
b) reconstruction de la répartition spatiale de l'absorption dans le premier volume partiel au moyen du premier algorithme de reconstruction,
c) reconstruction de la répartition spatiale de l'absorption dans le deuxième volume partiel au moyen du deuxième algorithme de reconstruction divergeant du premier.
